(19) Europäisches Patentamt
European Patent Office
Office européen des brevets

(11) Numéro de publication: 0 188 952 B1

# (12) FASCICULE DE BREVET EUROPÉEN

(45) Date de publication du fascicule du brevet: **12.10.88**

(51) Int. Cl.4: **C 07 C 69/63**, C 07 C 43/12

(21) Numéro de dépôt: **85402547.5**

(22) Date de dépôt: **18.12.85**

(54) **Télogènes fluorés à terminaison CC13, leur préparation et cotélomères biséquencés en dérivant.**

(30) Priorité: **26.12.84 FR 8419835**

(43) Date de publication de la demande: **30.07.86 Bulletin 86/31**

(45) Mention de la délivrance du brevet: **12.10.88 Bulletin 88/41**

(84) Etats contractants désignés: **AT BE CH DE GB IT LI LU NL SE**

(56) Documents cité:
**US-A-3 445 507**
**US-A-3 609 196**
**US-A-3 719 698**

(73) Titulaire: **ATOCHEM, 4 & 8, Cours Michelet La Défense 10, F-92800 Puteaux (FR)**

(72) Inventeur: **Boutevin, Bernard, Les Terres Blanches 1, rue Anselme Mathieu, F-34000 Montpellier (FR)**
Inventeur: **Pietrasanta, Yves, 14, Place Meril Pougade, F-34140 Meze (FR)**
Inventeur: **Lantz, André, Domaine de la Hêtraie, F-69390 Vernaison (FR)**

(74) Mandataire: **Leboulenger, Jean, ATOCHEM Département Propriété Industrielle, F-92091 Paris la Défense 10 Cédex 42 (FR)**

EP 0 188 952 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne de nouveaux télogènes fluorés à terminaison $CCl_3$ et leur emploi pour la fabrication de cotélomères biséquencés comportant une séquence totalement fluorée et une séquence polymérique compatible avec le millieu d'application.

Du fait de la faiblesse des forces d'interaction intermoléculaire, les composés organiques fluorés confèrent aux matériaux sur lesquels ils sont appliqués ou dans lesquels ils sont inclus, des propriétés particulièrement intéressantes, les principales étant des propriétés de surface (diminution de la tension superficielle critique), des propriétés de lubrification (diminution du coefficient de frottement), des propriétés de résistance aux agressions chimiques (dimiques de la mouillabilité du matériau) ou photochimiques (rayonnement divers) et des propriétés tensioactives exceptionnelles dans les milieux liquides organiques ou aqueux.

Cependant, cet apport énorme sur le plan des propriétés s'accompagne du problème de la miscibilité et de la compatibilité de ces composés fluorés avec les milieux d'application. Pour pallier cet inconvénient, une solution consiste à réaliser des composés biséquencés comportant d'une part, à une extrémité, une séquence fluorée de longueur et de nature variable, et, d'autre part, à l'autre extrémité, une séquence compatible avec le milieu d'application choisi, ces deux séquences pouvant être séparées entre elles par un certain nombre de groupements chimiques nécessaires, au cours de la synthèse, pour assurer leur liaison.

Selon la nature du milieu d'application, la séquence non fluorée pourra être par exemple, hydrophile (milieu aqueux) ou polymérique si le milieu d'application est lui-même un polymère, la séquance polymérique étant dans ce cas, soit de nature identique à celle du polymère, soit fortement compatibla avec lui. Ces composés biséquencés trouvent de nombreuses applications, notammant comme additifs pour mousses protéïniques, transport de gaz, tensioactifs,..

Différentes voies d'accès aux composés biséquencés comportant une séquence fluorée sont déjà connues.

On peut citer :

a) La condensation d'un composé comportant une chaîne fluorée plus ou moins longue et un groupe fonctionnel carboxylique sur une molécule hydrophile comportant une fonction alcool ou amine. Voir par exemple M.K. Bernett, Polymer. Eng. and Sci.,1977-17(7)450, qui décrit les composés suivants :

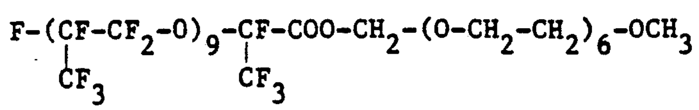

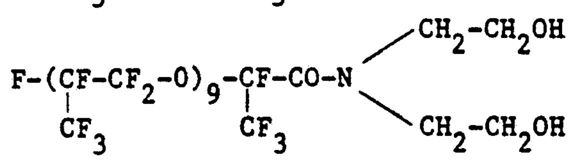

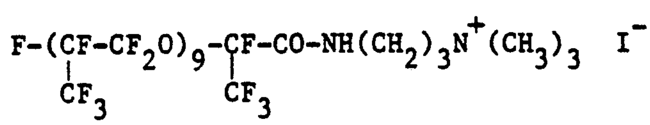

b) Télomérisation par voie radicalaire d'un monomère acrylique ou vinylique en présence d'un thiol fluoré agissant comme agent télogène. Voir, par exemple, le brevet EP-0 019 584 qui décrit des télomères biséquencés de formule :

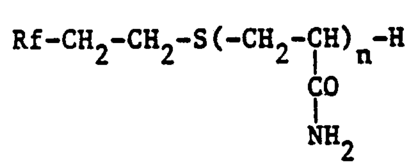

obtenus par une télomérisation radicalaire amorcée par l'azo-bisisobutyronitrile (AIBN) de l'acrylamide en présence du thiol $Rf-C_2H_4-SH$, Rf représentant un radical perfluoroalkyle.

c) Préparation séparée des deux séquences par télomérisation radicalaire (AIBN), puis condensation des deux télomères pour obtenir le composé biséquencé. Voir, par exemple, T. Hayashi, M. Matsuo et S. Kawakami (9ème Symposium International du Fluor, Avignon, Septembre 1977) qui décrivent la condeneation d'un télomére à extrêmité acide carboxylique obtenu à partir d'un acrylate d'alcool perfluoré et d'acide thioglycolique, avec un télomère terminé par une fonction alcool obtenu à partir d'un acrylate d'alkyle et de thioéthanol.

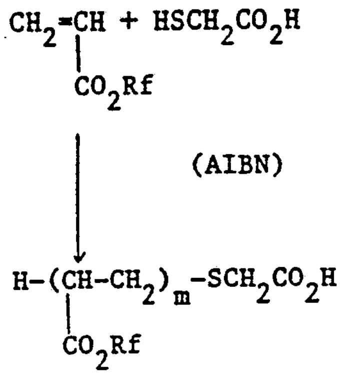


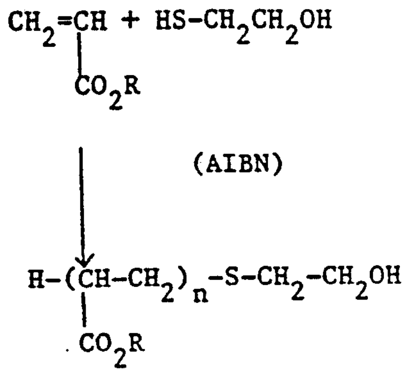


(I) (II)

(I) + (II) 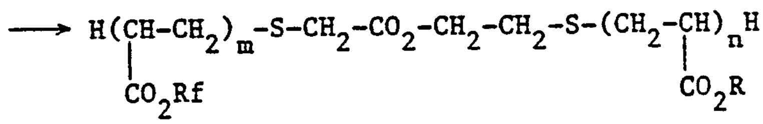

Cependant, les télomérisations par voie radicalaire ne sont pas facilement maitrisables et par conséquent ne permettent pas toujours d'obtenir de façon contrôlée et systématique des composés biséquencés parfaitement définis, ce qui nuit à la reproductibilité des résultats applicatifs.

Parmi les composés fluorés à groupement $CCl_3$ déjà connus, on peut citer l'ester $(CF_3)_2$ CH-OCO-$CCl_3$ décrit comme insecticide et nématocide dans le brevet US-3 445 507 et l'éther $CHF_2$-$CF_2$-O-$CCl_3$ décrit comme anesthésique dans le brevet US-3 609 196.

Il a maintenant été trouvé de nouveaux télogènes qui permettent, par télomérisation par catalyse rédox avec des monomères comportant une double liaison éthylénique, d'obtenir des cotélomères biséquencés comportant une séquence totalement fluorée et une séquence polymérique compatible avec le milieu d'application choisi.

Les nouveaux télogènes selon l'invention sont caractérisés par la présence d'un groupement terminal $CCl_3$ et peuvent être représentés par la formule générale :

$$Rf - Q - CCl_3 \qquad (III)$$

dans laquelle Rf désigne un radical perfluoroalkyle contenant de 2 à 20 atomes de carbone et Q un ensemble de groupements chimiques assurant la liaison.

Le radical perfluoroalkyle Rf qui contient de préférence de 2 à 14 atomes de carbone, peut être un radical linéaire $CF_3(CF_2)_m$ - (m = 1 à 19) ou un radical ramifié tel que, par exemple ceux de formules :

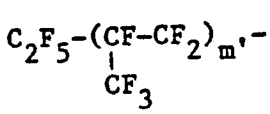 (où m' = 1 à 6)

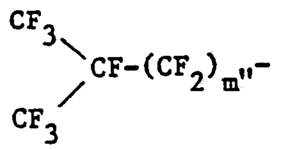 (où m'' = 1 à 17)

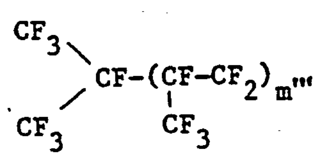 (où m''' = 1 à 5)

Le groupe de liaison Q dont la nature et la longueur dépendent essentiellement de la méthode utilisée pour la préparation du télogène (III), peut prendre notamment l'une des formes suivantes :

$-(CH_2)_r-OCO-(CH_2)_s-$ (IV)

$-(CH_2)_r-OCO-(CH_2)_s-CH(X)-W-$ (V)

$-(CH_2)_{r'}-SO2-N(R)-CH_2CH_2-OCO-(CH_2)_s-$ (VI)

$-(CH_2)_{r'}-SO_2-N(R)-CH_2CH_2-OCO-(CH_2)_s-CH(X)-W-$ (VII)

$-(CH_2)_{r'}-COO-(CH_2)_{s'}-$ (VIII)

$-(CH_2)_{r'}-COO-(CH_2)_{s'}-CH(X)-W-$ (IX)

$-(CH_2)_{r'}-COO-CH_2-CH(OH)-CH_2-$ (X)

$-(CH_2)_{r'}-CH(X)-W-$ (XI)

$-(CH_2)_{r'}-CONH-(CH_2)_{s'}-CH(X)-W-$ (XII)

$-(CH_2)_{r'}-SO_2NH-(CH_2)_{s'}-CH(X)-W-$ (XIII)

$-(CH_2)_r-Y-Z-CH(X)-W-$ (XIV)

$-O-CF(X)-CF_2-$ (XV)

dans lesquelles r est un nombre entier allant de 1 à 12, de préférence égal à 1 ou 2, r' est un nombre entier allant de 0 à 12, de préférence égal à 0 ou 2, s est un nombre entier allant de 0 à 12, s'est un nombre entier allant de 1 à 12, X représente un atome d'hydrogène de chlore ou de brome, W représente un pont $-CH_2-$, $-CH_2-CCl_2-$, $-CH_2-CCl_2-CH_2-$ ou $-CH_2-CCl_2-CF_2-$, R représente un atome d'hydrogène ou un groupe alkyle contenant de 1 à 4 atomes de carbone, Y représente un atome d'oxygène ou de soufre et Z représente un pount $-CH_2-$, $-CH_2-C_6H_4-$ ou $-COO-(CH_2)_t-$, t étant un nombre entier allant de 0 à 9, de préférence égal à 0 ou 1.

Selon l'invention Rf et Q peuvent également prendre des formes 10 plus complexes telles que les suivantes :

$X-[CF(ORf)-CF_2]_u-W'-$ (XVI)

$X-[CR'(CO-O-(CH_2)_r-Rf)-CH_2]_u-W'-$ (XVII)

dans lesquelles Rf, X et r ont les mêmes significations que ci-dessus, u est un nombre allant de 2 à 100, R' représente un atome d'hydrogène, de chlore ou de fluor ou un groupe méthyle et W' représente une liaison

directe ou un pont $-CCl_2-$, $-CCl_2-CH_2-$ ou $-CCl_2-CF_2-$.

Les télogènes selon l'invention peuvent être préparés de différentes façons qui tendent toutes à fixer plus ou moins directement la terminaison $CCl_3$ sur un radical fluoré Rf.

Selon une première méthode qui permet d'accéder aux télogènes (III) dans lesquels Q correspond à l'une des formules (IV) à (X), on estérifie un alcool fluoré de formule :

$$Rf-(CH_2)_r-OH \qquad (XIX)$$

ou

$$Rf-(CH_2)_{r'}-SO_2-\underset{\displaystyle R}{\underset{|}{N}}-CH_2CH_2-OH \qquad (XX)$$

au moyen d'un acide carboxylique du type :

$$HOOC-(CH_2)_s-CCl_3 \qquad \text{ou} \qquad HOOC-(CH_2)_s-\underset{\displaystyle X}{\underset{|}{CH}}-W-CCl_3$$

(XXI) (XXII)

ou inversement, on estérifie un acide carboxylique fluoré de formule:

$$Rf-(CH_2)_{r'}-COOH \qquad (XXIII)$$

au moyen d'un alcool de formule:

$$HO-(CH_2)_{s'}-CCl_3 \qquad \text{ou} \qquad HO-(CH_2)_{s'}-\underset{\displaystyle X}{\underset{|}{CH}}-W-CCl_3$$

(XXIV) (XXV)

ou au moyen d'un époxyde de formule

$$\underset{\diagdown\ O\ \diagup}{H_2C-CH}-CH_2-CCl_3 \qquad (XXVI)$$

La réaction d'estérification peut être effectueé dans les conditions habituelles connues en présence d'un catalyseur acide tel que, par exemple, l'acide p-toluène sulfonique, et dans un solvant organique comme le chloroforme, le dichlorométhane, le toluène, le benzène ou le dioxanne.

Une seconde méthode qui permet d'accéder aux télogènes (III) dans lesquels Q correspond à l'une des formules précédentes (V), (IX) et (XI) à (XV) consiste en la monoaddition d'un halogéno-alcane tel que le tétrachlorure de carbone, le bromotrichlorométhane, le chloroforme, l'hexachloroéthane, le pentachloroéthane, l'hexachloro-1,1,1,3,3,3 propane et le difluoro-2,2 hexachloro-1,1,1,3,3,3 propane, sur une oléfine fluorée telle que celles de formules :

$Rf-(CH_2)_r-OCO-(CH_2)_s-CH = CH_2$ (XXVII)

$Rf-(CH_2)_{r'}-COO-(CH_2)_{s'}-CH = CH_2$ (XXVIII)

$Rf-(CH_2)_{r'}-CH = CH_2$ (XXIX)

$Rf-(CH_2)_{r'}-CONH-(CH_2)_{s'}-CH = CH_2$ (XXX)

$Rf-(CH_2)_{r'}-SO_2NH-(CH_2)_{s'}-CH = CH_2$ (XXXI)

$Rf-(CH_2)_r-Y-Z-CH = CH_2$ (XXXII)

$Rf-O-CF = CF_2$ (XXXIII)

Cette monoaddition peut être réalisée de façon connue en soi par voie radicalaire ou par catalyse rédox dans un solvant inerte, de préférence un nitrile comme l'acétonitrile, le butyronitrile et l'isobutyronitrile. On opère généralement à une température comprise entre 60 et 130°C, de préférence entre 80 et 100°C. Par voie

radicalaire, on opère en présence des amorceurs habituels : composés diazoïques comme l'azobisisobutyronitrile, peroxydes comme le peroxyde de benzoyle ou de tert.-butyle, hydroperoxydes comme l'hydroperoxyde de tert.-butyle, ou percarbonates. Pour la catalyse rédox, on préfère opérer en présence d'un sel de cuivre ou d'un mélange chlorure ferrique-benzoïne.

Une autre méthode pour accéder aux télogènes (III) dans lesquels Q correspond aux formule (XII) et (XIII) consiste à condenser un halogénure d'acide carboxylique ou sulfonique fluoré, par exemple :

$$Rf-(CH_2)_{r'}-COCl \text{ (ou } -SO_2Cl) \qquad (XXXIV)$$

avec une amine de formule :

$$H_2N-(CH_2)_{s'}-\underset{\displaystyle X}{\underset{|}{CH}}-W-CCl_3 \qquad (XXXV)$$

dans les conditions bien connues pour ce type de réaction.

Les télogènes du type :

$$Rf-(CH_2)_r-Y-CH_2-C_6H_4-\underset{\displaystyle X}{\underset{|}{CH}}-W-CCl_3 \qquad (XXXVI)$$

peuvent également être préperés par condensation d'un alcool ou thiol fluoré : $Rf-(CH_2)_r-YH$ avec un composé de formule :

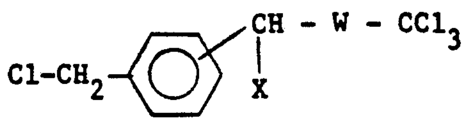

(XXXVII)

qu'on peut obtenir par monoaddition d'un halogéno-alcane tel que ceux précédemment cités sur le chlorométhyletyrène.

Enfin, les télogènes selon l'invention dans lesquels Rf-Q-présente la formule (XVI) ou (XVII) peuvent être obtenus par télomérisation d'une oléfine fluorée de formule :

$$Rf-O-CF = CF_2 \qquad (XXXIII)$$

$$Rf-(CH_2)_r-OCO-\underset{\displaystyle R'}{\underset{|}{C}} = CH_2 \qquad (XXXVIII)$$

sur un halogéno-alcane tel que ceux précédemment cités.

Cette télomérisation peut être réalisée par voie radicalaire ou par catalyse rédox.

La plupart des composés formules (XIX) à (XXXV) sont des produits connus ou faciles à préparer par des méthodes connues pour des composés analogues. On pourra se référer, par exemple, aux brevets et publications suivantes :

(XIX) : Brevets FR-1 380 579, US-2 259 628, US-2 666 797, US-3 016 407, US-3 083 224 et DF-1 132 907; Eur. Polym. J. 1976, 12, 219.

(XX) : Brevets FR-2 034 142 et US-2 803 656.

(XXI) : M. BELBACHIR et al, Makrom. Chem. 1982, 183, 2347 - 2358.

(XXII) : B. BOUTEVIN et al, Eur. Polym. J. 1977, 13, 935; B. BOUTEVIN et al, Makrom. Chem. 1981, 182, 2927; M. BELBACHIR et al, Makrom. Chem. 1982, 183, 2347; A. BATTAIS et al, Makrom. Chem. 1982, 183, 2359.

(XXIII) : N.O. BRACE, J. Org. Chem. 1962, 27, 4451 - 4498; brevet FR-1 343 601.

(XXIV) : B BOUTEVIN et al, Eur. Polym. J. 1978, 14, 353; M. BELBACHIR et al, Makrom. Chem. 1982, 183, 2347

(XXV) : F.M. LEWIS et al, J. Am. Chem. Soc. 1954, 76, 457; B. BOUTEVIN et al, Eur. Polym. J. 1977, 13, 935; B. BOUTEVIN et al, Makrom. Chem. 1981, 182, 2927; B. BOUTEVIN, et al Makrom. Chem.

1983, 184, 2401; A. BATTAIS et al, Makrom. Chem. 1982, 183, 2359.

(XXVI) : B. BOUTEVIN et al, J. of Fluorine Chem. 1981, 17, 357.

(XXVII) : Brevet FR-1 327 328

(XXVIII) : Brevet FR-1 140 072

(XXIX) : R.N. HASZELDINE, J.C.S. 1952, 3483 - 3498.

(XXX) : Brevets FR-1 558 735, GB-2 017 774 et US-3 470 130

(XXXI) : Brevets GB-2 017 774 et US-3 442 664.

(XXXII) : Brevet GB-869 343

(XXXIII) : S.P. SHRLAKYAN et al, Izvest. Akad. Nauk. SSSR 155, 1965; J. Polym. Sci. 1970, 8, 1091 - 98

(XXXIV) : Brevets FR-1 600 425, FR-2 374 287, FR-1 341 601, US-2 593 737 et US-2 732 398.

(XXXV) : B. BOUTEVIN et al, Eur. Polym. J. 1977, 13, 935.

Les oléfines fluorées de formule (XXXII) dont certaines seulement sont connues (brevet GB-869 343) peuvent être préparées en faisant réagir un chlorure de formule :

$$Cl\text{-}Z\text{-}CH = CH_2 \qquad \text{(XXXIX)}$$

tel que, par exemple, le chlorure d'allyle, le chlorométhylstyrène, le chloroformiate de vinyle et le chloroformiate d'allyle, sur un alcool fluoré (XIX) ou le thiol correspondant, par catalyse par transfert de phase en présence d'hydrogénosulfate de tétrabutylammonium.

Les télogènes (III) selon l'invention peuvent être utilisés pour fabriquer des cotélomères biséquencés comportant une séquence totalement ou partiellement fluorée et une séquence polymérique compatible avec le milieu d'application désiré. Ces cotélomères qui peuvent être représentés par la formule générale :

$$Rf\text{-}Q\text{-}CCl_2\text{-}(CH_2\text{-}\underset{R_2}{\overset{R_1}{\overset{|}{\underset{|}{C}}}})_w\text{-}Cl \qquad \text{(XXXX)}$$

sont des produits nouveaux et, en tant que tels, font partie de la présente invention.

Dans la formule ci-dessus, Rf et Q ont la même signification que précédemment, w désigne le degré de télomérisation et peut prendre toute valeur allant de 1 à 1500 et de préférence comprise entre 1 et 300, $R_1$ représente un atome d'hydrogène, de chlore ou de fluor ou un groupe cyano, méthyle ou carboxyméthyle, $R_2$ représente un atome d'halogène (Cl, Br, F), un groupe hydroxy, cyano, carboxy, ester carboxylique, alcoxyalkyle, sulfo ou carbamoyle, ou un radical pyrrolidone-2 yle-1, pyridyle-4 ou phényle éventuellement substitué par un groupe alkyle ($C_1$-$C_4$), chlorométhyle ou sulfo, $R_1$ ne peut être un groupe carboxyméthyle que si $R_2$ est un groupe carboxy, et $R_1$ et $R_2$ ne peuvent pas représenter simultanément un groupe cyano.

Quand $R_2$ représente un groupe ester carboxylique, celui-ci peut dériver d'un alcool aliphatique contenant de 1 à 18 atomes de carbone, de l'éthylène ou du propylène glycol ou d'un poly (1 à 12)-oxyéthylène et/ou propylène-glycol. Par groupe alcoxyalkyle, on entend ici un groupe $-(CH_2)_x$-O-alkyl, le radical alkyle contenant de 1 à 4 atomes de carbone et x désignant un nombre entier de 1 à 12. Quand $R_2$ représente ou contient un groupe sulfo, celui-ci peut être sous la forme d'un sel métallique, en particulier un sel de métal alcalin. Le groupe carbamoyle que peut représenter $R_2$ peut être non substitué ou substitué par un ou deux groupes alkyle ($C_1$-$C_4$), hydroxyalkyle ($C_1$-$C_4$) ou alcoxyalkyle ($C_2$ - $C_8$).

Conformément à la présente invention, on obtient les cotélomères (XXXX) à partir des télogènes (III) par télomérisation par catalyse rédox (B BOUTEVIN et al., J. Polym. Sci. 1981 - 10, p. 499 - 509) avec un ou plusieurs monomères éthyléniques de formule :

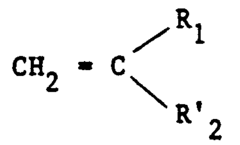

(XXXXI)

dans laquelle $R_1$ est tel que défini ci-dessus et $R'_2$ a la même signification que $R_2$, à ceci près que le groupe hydroxy est remplacé par un groupe acyloxy dérivé d'un acide aliphatique carboxylique contenant de 2 à 5 atomes de carbone.

Comme exemples de monomères éthyléniques de formulé (XXXXI), on peut citer le chlorure, fluorure ou bromure de vinyle, le chlorure ou fluorure de vinylidène, le styrène, le vinyltoluène, l'α-méthylstyrène, le p-chlorométhyl-styrène, les acides acrylique, méthacrylique, itaconique, α-chloro-acrylique, α-fluoro-acrylique et α-cyano-acrylique et leurs esters comme l'acrylate ou le méthacrylate de méthyle, de butyle, d'isobutyle, d'hexyle, d'heptyle, d'éthyl-2 hexyle, de lauryle ou de stéaryle, le mono-acrylate ou méthacrylate d'éthylèneglycol, de propylèneglycol ou de poly-oxyéthylène (et/ou propylène)glycols, l'acrylonitrile, le méthacrylonitrile, le chloro-2 acrylonitrile, les acides vinylsulfonique et styrène-p-sulfonique et leurs sels alcalins, l'acrylamide, le méthacrylamide, le N-isopropyl-acrylamide, les N-(hydroxyméthyl)acrylamides et méthacrylamides, les N-(alcoxyméthyl)acrylamides et méthacrylamides, la N-vinyl-pyrolidone-2, la vinyl-4 pyridine, l'acétate de vinyle, le propionate de vinyle, l'isobutyrate de vinyle.

Les cotélomères selon l'invention peuvent être utilisés dans de nombreux domaines, en particulier dans tous les cas où on cherche à abaisser la tension superficielle d'un liquide ou à conférer à un substrat un effet hydrophobe et oléophobe. Ils peuvent par exemple être utilisés comme mouillants, agents moussants, émulsifiants, dispersants, agents d'étalement de cires, vernis et peintures, additifs aux lubrifiants ou aux émulseurs employés pour la lutte contre l'incendie.

Les exemples suivants illustrent l'invention sans la limiter.

**EXEMPLE 1 :** Préparation de $C_6F_{13}$-$CH_2CH_2$-O-CO-CHCl-$CH_2$-$CCl_3$

Dans un ballon muni d'un réfrigérant, on introduit 100 g (0,2392 mole) d'acrylate de perfluorohexyl-2 éthyle, 55,25 g (0,3588 mole) de tétrachlorure de carbone, 0,4761 g ($4{,}81.10^{-3}$ mole) de chlorure cuivreux et 150 ml de butyronitrile. On porte ensuite le mélange à 120°C sous reflux. Après 26 heures de réaction, on verse le mélange dans une ampoule à décanter et le lave trois fois avec 100 ml d'une solution aqueuse d'acide chlorhydrique à 10 % pour détruire le catalyseur, puis une fois à l'eau. On extrait ensuite les eaux-mères avec trois fois 100 ml d'éther anhydre, puis on réunit les phases organiques et les sèche sur sulfate de sodium. Après filtration, on chasse alors les solvants au rotavapor et obtient ainsi 102,2 g d'un produit brut que l'on distille sous vide ; le tétrachloro-2,4,4,4 butyrate de perfluorohexyl-2 éthyle passe en tête de colonne entre 97 et 100° sous un vide de 0,01 torr.

En RMN du $^1H$, l'acrylate fluoré de départ se présente sous le forme d'un système ABX pour la partie $CH_2$ = CH-CO-.

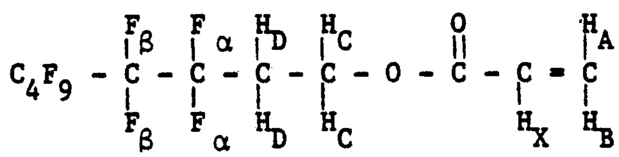

Les protons $H_C$ ET $H_D$ se présentent sous la forme d'un système $A_2X_2$. Les protons $H_C$ donnent un triplet centré à $4{,}45.10^{-6}$, où $J_{CD}$ = 6,6Hz. Les protons $H_D$ centrés à $2{,}55.10^{-6}$ sortent sous forme d'un triplet où $J_{DC}$ = 6,6 Hz. Un étalement du spectre entre 2 et 3 ppm montre que le triplet des protons $H_D$ est détriplé par les fluors en α où $J_{D\alpha}$ = 18 Hz, détriplé par les fluors en β où $J_{D\beta}$ = 1,5 Hz, ce qui donne 27 signaux au total.

Le spectre RMN $^1H$ du tetrachloro-2,4,4,4 butyrate de perfluoro-hexyl-2 éthyle :

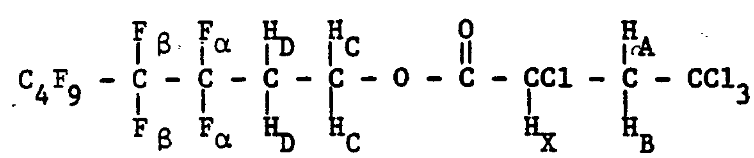

se présente sous une forme similaire à celle de l'acrylate de départ. Cependant, le système ABX est blindé : on trouve la partie AB à $3{,}28.10^{-6}$ alors que celle de l'acrylate de depart est centreé à $6{,}25.10^{-6}$. La partie X est aussi blindée à $3{,}2.10^{-6}$, la partie X de l'acrylate de départ étant centrée à $5{,}85.10^{-6}$. On observe un glissement

chimique de 2,45.$10^{-6}$ pour la partie X.

Dans le tableau 1 ci-dessous sont répertoriées les différentes constantes de couplage pour l'acrylate de départ et le tétrachloro-2,4,4,4 butyrate.

**TABLEAU 1**

| Constantes de couplage | $C_6F_{13}C_2H_4$-OCO-CH = $CH_2$ | $C_6F_{13}C_2H_4$-OCO-CHCl-$CH_2$-$CCl_3$ |
|---|---|---|
| $V_A.10^6$ | 6,4 | 3,8 |
| $V_B.10^6$ | 6,1 | 3,2 |
| $J_{AB}$ en Hz | 16,9 | 15,35 |
| $J_{AX}$ en Hz | 10,65 | 8,14 |
| $J_{BX}$ en Hz | 2,15 | 3,46 |
| $J_{CD}$ en Hz | 6,6 | 6,6 |
| $J_{D\alpha}$ en Hz | 18 | 18 |
| $J_{D\beta}$ en Hz | 1,5 | 1,5 |

**EXEMPLE 2:** Estérification de l'alcool fluoré $C_6F_{13}CH_2CH_2OH$ par l'acide trichloroacétique

Dans un ballon monocol muni d'un réfrigérant et d'un Dean Stark, on introduit 110 g d'alcool fluoré (0,3022 mole), 100 g d'acide trichloroacétique (0,6012 mole), 300 ml de benzène comme solvant et 0,4 g d'acide paratoluène-sulfonique. Le mélange est porté à reflux à 85°C pendant 6 heures. On verse ensuite le mélange réactionnel dans une ampoule à décanter et extrait d'abord l'excès d'acide trichloroacétique par trois fractions de 100 ml d'eau permutée. Dans un second temps, on neutralise l'acide restant dans la phase organique; pour cela, on extrait avec trois fois 100 ml d'une solution aqueuse à 10 % de carbonate de sodium en contrôlant le pH. On extrait ensuite avec de l'éther anhydre la phase organique des eaux mères (3 fois 70 ml d'éther), puis on filtre, sèche sur sulfate de sodium et chasse les solvants au rotavapor.

L'ester brut ainsi obtenu est ensuite purifié par distillation sous vide (0,01 torr) dans un bain d'huile thermostaté à 100°C. Dans ces conditions, le trichloroacétate de perfluorohexyl-2 éthyle passe en tête de colonne à 50°C. On en obtient 135 g, soit un rendement de 88 %.

En RMN du $^1H$, l'alcool fluoré de départ:

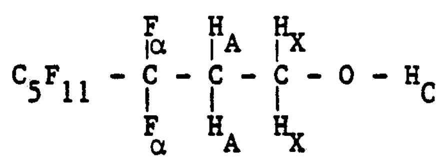

se présente sous la forme d'un système AX où les deux protons $H_X$ résonnent sous forme d'un triplet centré 3,9.$10^{-6}$. Les protons $H_A$ sont détriplés par les protons $H_X$ et détriplés par les deux fluors en α. On trouve au total 9 signaux centrés à 2,35.$10^{-6}$. L'hydrogène de l'alcool $H_C$ quant à lui, sort sous forme d'un singulet à 3,45.$10^{-6}$.

Pour l'ester, la RMN du $^1H$ montre sans ambiguité la structure suivante :

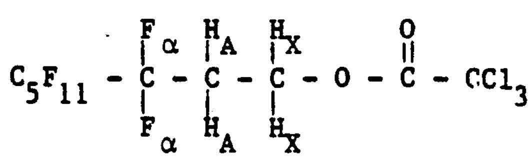

En effet, le singulet précédemment centré à 3,45.$10^{-6}$ a totalement disparu. Les deux protons $H_X$ sortent sous forme d'un triplet centré à 4,65.$10^{-6}$ (ce déblindage confirme également la formation de l'ester). Les deux autres protons $H_A$ résonnent sous forme de 9 signaux centrés à 2,55.$10^{-6}$.

**EXEMPLE 3:** Préparation de $C_6F_{13}$-$CH_2CH_2$-O-CO-CHCl-$CH_2$-$CCl_3$

On opère comme à l'exemple 2, mais en remplaçant l'acide trichloroacétique par la même quantité molaire d'acide tétrachloro-2,4,4,4 butanoïque préparé par télomérisation de l'acide acrylique avec le tétrachlorure de carbone suivant le procédé décrit par BOUTEVIN et al, Tetrahedron Letters 1977, 49, 4315.

On obtient avec un rendement quantitatif le même ester qu'à l'exemple 1.

**EXEMPLE 4:** Préparation de $C_6F_{13}$-$CH_2CH_2$-O-CO-$CH_2$-CHCl-$CH_2$-$CCl_3$

On opère comme à l'exemple 2, mais en remplacant l'acide trichloroacétique par l'acide tétrachloro-3,5,5,5 pentanoïque préparé par télomérisation de l'acide vinylacétique avec le tétrachlorure de carbone suivant le procédé décrit par BOUTEVIN et al, Makromolecular Chemie 1981, 182, 2927.

On obtient avec un rendement quantitatif le tétrachloro-3,5,5,5 pentanoate de perfluorohexyl-2 éthyle dont la structure est determineé par RMN du $^1H$ :

- les deux $CH_2$ en α du groupement CHCl résonnent à $3,20.10^{-6}$ sous la forme d'un massif complexe (faux doublet),
- le groupement -CHCl- à $4,85.10^{-6}$ donne un multiplet,
- à $2,52.10^{-6}$ on trouve le $CH_2$ en α du $CF_2$, et
- à $4,23.10^{-6}$ on trouve un triplet du -$CH_2$- en α du groupement ester.

**EXEMPLE 5:** Préparation de $C_6F_{13}$-$CH_2CH_2$-CO-O-$CH_2$-CHCl-$CH_2$-$CCl_3$

En opérant comme à l'exemple 2 à partir de l'acide $C_6F_{13}$-$CH_2CH_2$-$CO_2H$ et du tétrachloro-2,4,4,4 butanol, on obtient avec un rendement de 95 % le perfluorohexyl-3 propionate de tétrachloro-2,4,4,4 butyle dont le spectre RMN$^1$H presente trois séries de pics:

- à $3,23.10^{-6}$, un faux doublet (2 protons) dû au $CH_2$ en α de $CCl_3$,
- entre 4,2 et $4,7.10^{-6}$, un multiplet (3 protons du CHCl et du $CH_2$ en α de l'oxygène),
- entre 2,2 et $2,7.10^{-6}$, un multiplet (4 protons) des deux groupements méthylène de l'acide fluoré de départ.

Le tétrachloro-2,4,4,4 butanol a été préparé par addition du tétrachlorure de carbone sur l'alcool allylique suivant le procédé décrit par BOUTEVIN et al, European Polymer J. 1977, 13, 935.

**EXEMPLE 6:** Préparation de $C_6F_{13}$-$CH_2CH_2$-CO-O-$CH_2$
CH-$CH_2$-$CCl_3$
|
OH

On fait réagir au reflux pendant 5 heures une mole d'acide $C_6F_{13}$-$CH_2CH_2$-$CO_2H$ et une mole de trichloro-4,4,4 époxy-1,2 butane dans 100 ml d'acétonitrile en présence de 0,1 mole de diéthyl lauryl amine comme catalyseur. Après évaporation de l'acétonitrile et dilution à l'éther, on traite le mélange par de l'eau carbonatée, puis sèche et évapore.

On obtient ainsi avec un rendement de 80 % le perfluorohexyl-3 propionate d'hydroxy-2 trichloro-4,4,4 butyle pur (absence des produits de départ en chromatographie en phase vapeur).

En I.R., on trouve à 1720 $cm^{-1}$ l'absorption caractéristique des carbonyl esters et entre 1000 et 1200 $cm^{-1}$ les absorptions des liaisons CF et -C-O-C- αβ insaturée. De plus, à 3400 $cm^{-1}$, on observe les vibrations du groupement hydroxyle qui démontrent l'ouverture des cycles époxydiques.

| Analyse centésimale | F % | Cl % |
|---|---|---|
| Calculé pour $C_{13}F_{13}O_3Cl_3H_{10}$ | 43,52 | 18,77 |
| Trouvé | 42,8 | 19 |

Le trichloro-4,4,4 époxy-1,2 butane a été préparé suivant la méthode de BOUTEVIN et al, J. of Fluorine Chem. 1981, 17, 357.

**EXEMPLE 7:** Préparation de $C_6F_{13}$-$CH_2CH_2$-O-$CH_2$-CHCl-$CH_2$-$CCl_3$

1. Dans un ballon muni d'un réfrigérant, on introduit 0,1 mole d'allyloxy-1 perfluorohexyl-2 éthane, 0,1 mole de tétrachlorure de carbone, $1.10^{-3}$ mole de chlorure ferrique, $1.10^{-3}$ mole de benzoïne comme réducteur et 10 ml d'acétonitrile. On chauffe le mélange à l'ébullition (82°C) et suit l'avancement de la réaction par chromatographie en phase vapeur. Au bout de 48 heures, on dilue le mélange réactionnel avec du tétrachlorure de carbone et le lave plusieurs fois avec une solution d'acide chlorhydrique à 10 % pour éliminer les sels de fer, puis avec de l'eau carbonatée. Après séchage et élimination des solvants, le résidu est distillé sous vide. On isole ainsi avec un rendement de 70 % l'éther

$C_6F_{13}$-$CH_2CH_2$-O-$CH_2$-CHCl-$CH_2CCl_3$ (Eb 0,05 torr = 86°C).

Le spectre RMN $^1$H de cet éther :

| $C_6F_{13}$ - | $CH_2$ - | $CH_2$ - O - | $CH_2$ - | CHCl - | $CH_2$ - $CCl_3$ |
|---|---|---|---|---|---|
| | (a) | ($b_2$) | ($b_1$) | (d) | (c) |

présente les divers signaux suivants :

- Un multiplet à $2,4.10^{-6}$ attribué aux deux protons (a): le système complexe obtenu peut être résolu en prenant en compte les couplages avec les protons ($b_2$) (triplet: J = 7 Hz) de deux atomes de fluor (en α ou en β ) (triplet, J = 18,5 Hz) et de deux autres atomes de fluor (en α ou en β) (triplet, J = 1,5 Hz);
- Un signal à $3,85.10^{-6}$ correspondant aux deux protons ($b_2$) qui ne sont couplés qu'avec les deux protons (a) (apparaissant comme équivalents) pour donner un triplet de constante de couplage égale à 7 Hz ;
- Le signal à $3,85.10^{-6}$ est partiellement confondu avec le multiplet des protons ($b_1$) qui forment avec le proton (d) (partie X centrée vers 4 $35.10^{-6}$) et les protons (c) (entre 2,9 et $3,6.10^{-6}$) un système complexe de type AA'BB'X.

Le spectre RMN $^{13}C$ est également confondu à la structure proposée. On trouve en effet le carbone trichlorométhyle à $97,1.10^{-6}$ (J = 5 Hz), le carbone ($b_1$) à $74,6.10^{-6}$, le carbone ($b_2$) légèrement détriplé à $63,7.10^{-6}$, les carbones (c) et (d) à 59,2 et $58,9.10^{-6}$ et le carbone (a) triplet (J = 21,6 Hz) à $31,8.10^{-6}$. Les carbones fluorés très découplés donnent un ensemble de raies très peu intenses entre 90 et $130.10^{-6}$.

En RMN $^{19}F$ (référence interne $CFCl_3$) on trouve les fluors (a) à $82,3.10^{-6}$, les fluors (f) à $114,7.10^{-6}$ et les fluors (b) à $128.10^{-6}$:

$CF_3$ - $CF_2$ - $CF_2$ - $CF_2$ - $CF_2$ - $CF_2$ - $CH_2$ - $CH_2$ - O -
(a) (b) (c) (d) (e) (f)

Les fluors (c), (d) et (e) donnent trois pics à 123,3; 124,7 et $125,3.10^{-6}$.

2. L'allyloxy-1 perfluorohexyl-2 éthane utilisé dans cet exemple peut être obtenu de la façon suivante :

Dans un ballon à deux tubulures muni d'un réfrigérant et équipé d'une agitation mécanique tournant à 500 tours/mn, on maintient à 42°C pendant 6 heures un mélange comprenant 0,0769 mole d'alcool $C_6F_{13}$-$CH_2$-$CH_2OH$, 80 ml d'une solution de soude à 50 %, 2,6 g ($7,69.10^{-3}$ mole) d'hydrogénosulfate de tétrabutylammonium (TBAH) et 0,3845 mole de chlorure d'allyle. A la fin de la réaction, le mélange réactionnel est dilué avec 20 ml de chlorure de méthylène et lavé plusieurs fois à l'eau pour éliminer le TBAH. Le solvant est ensuite évaporé et le résidu distillé sous vide. L'allyloxy-1 perfluorohexyl-2 éthane bout à 76°C sous 20 torr. Le rendement atteint 95 % en produit distillé à 95 % de pureté.

Le spectre de RMN$^1$H présente bien le système ABX complexe des protons de la double liaison allylique entre 5,0 et $6,1.10^{-6}$. On y observe également un doublet détriplé à $4,0.10^{-6}$ et un triplet à $3,7.10^{-6}$ ainsi qu'un triplet deux fois détriplé à $2,4.10^{-6}$.

En opérant de la même façon à partir des intermédiairee fluorés suivants :

. $C_7F_{15}$-$CH_2$-OH
. $C_6F_{13}$-$CH_2CH_2$-SH

ou en remplaçant le chlorure d'allyle par le p-chlorométhylstyrène, on obtient les éthers suivants :

. $C_7F_{15}$-$CH_2$-O-$CH_2$-CH = $CH_2$
. $C_6F_{13}$-$CH_2$-$CH_2$-S-$CH_2$-CH = $CH_2$
. $C_6F_{13}$-$CH_2CH_2$-O-$CH_2$-$C_6H_4$-CH = $CH_2$

qu'on peut ensuite faire réagir avec $CCl_4$ comme à l'exemple 7.1

**EXEMPLE 8:** Préparation de $C_6F_{13}$-$CH_2CH_2$-O-$CH_2$-$C_6H_4$-CHCl-$CH_2$-$CCl_3$

On mélange 0,1 mole de l'éther $C_6F_{13}$-$CH_2CH_2$-O-$CH_2$-$C_6H_4$-CH= $CH_2$, 0,5 mole de tétrachlorure de carbone, 0,001 mole de chlorure cuivreux et 50 ml d'acétonitrile dans un tube que l'on scelle. Après 10 heures de réaction à 120°C, on ouvre le tube, évapore l'acétonitrile et l'excès de tétrachlorure de carbone et lave à l'eau acidulée le produit de la réaction. Après filtration sur colonne de silice, on obtient 47,5 g du composé désiré, soit un rendement de 75 %.

En RMN$^1$H, on observe six pics :

- le -$CH_2$- en $_\alpha$ du $CCl_3$ à $3,50.10^{-6}$ (système AB de l'ABX)
- le -CHCl- en α du noyau aromatique à $5,21.10^{-6}$ (faux triplet)
- les quatre protons aromatiques à $7,25.10^{-6}$
- les $CH_2$ en α du noyau aromatique, singulet à $4,5.10^{-6}$
- le $CH_2$ en α de l'oxygéne à $3,75.10^{-6}$
- le $CH_2$ en α du $CF_2$ à $2,45.10^{-6}$ (triplet, détriplet).

**EXEMPLE 9:**

On opère comme à l'exemple 8 à partir de 0,1 mole d'allyloxy-1 perfluorohexyl-2 éthane, 0,3 mole de difluoro-2,2 hexachloro-1,1,1,3,3,3 propane, 0,005 mole d'un mélange équimoléculaire de chlorure ferrique et de benzoïne et 50 ml d'acétonitrile. On chauffe à 110°C pendant 50 heures.

On obtient avec un rendement de 80 % l'adduct de formule :
$C_6F_{13}$-$CH_2CH_2$-O-$CH_2$-CHCl-$CH_2$-$CCl_2$-$CF_2$-$CCl_3$
Si on remplace le difluoro-hexachloro-propane par le tétrachlorure de carbone, on obtient dans les mêmes conditions le même éther qu'à l'exemple 7.1 avec un rendement de 92 %.
Les exemples 10 à 13 suivants illustrent la préparation de cotélomères bisequencés à partir des télogènes selon l'invention.

**EXEMPLE 10:**

Dans un ballon monocol muni d'un réfrigérant, on introduit 2 g (0,0035 mole) du tétrachloro-2,4,4,4 butyrate de perfluorohexyl-2 éthyle obtenu à l'exemple 1, 2,485 g (0,035 mole) d'acrylamide, 0,0468 g de chlorure ferrique, 0,0614 g de benzoïne et 30 ml de butyronitrile. On immerge le ballon dans un bain d'huile thermostaté à 125°C pendant 16 heures et demie; le cotélomère précipite dans le milieu réactionnel. On lave ensuite le précipité trois fois à l'acétone pour éliminer l'acrylamide et l'ester n'ayant pas réagi, puis trois fois à l'éther anhydre. Après filtration et séchage à 80°C sous un vide de 20 torr, on obtient 1,1933 g de cotélomère contenant 11,84 % de fluor, soit un degré de télomérisation moyen en nombre $\bar{D}\bar{P}\bar{n}$ de 21,33 calculé par la relation suivante :

$$\bar{D}\bar{P}\bar{n} = \frac{347,90}{\%\,F} - 8,05$$

**EXEMPLE 11:**

On opère comme à l'exemple 10, mais en augmentant d'un tiers les quantités de chlorure ferrique et de benzoïne. On obtient 0,8749 g d'un cotélomère contenant 13,23 % de fluor, ce qui correspond à un degré de télomérisation moyen de 18,24 (relation précédente).

**EXEMPLE 12:**

On effectue huit opérations de télomérisation de l'ester $C_6F_{13}$-$CH_2CH_2$-OCO-$CCl_3$ avec l'acrylamide en utilisant des quantités variables de catalyseur (mélange équimolaire de $FeCl_3$ et de benzoïne) et en procédant comme suit :
Dans un ballon de 100 ml, on introduit 7 g (0,09859 mole) d'acrylamide et 5 g (0,009859 mole) du trichloroacétate de perfluorohexyl-2 éthyle obtenu à l'exemple 2. On ajoute ensuite 40 ml de butyronitrile contenant une quantité de catalyseur telle que le rapport molaire (Co): $FeCl_3$/acrylamide prenne la valeur indiquée au tableau 2 ci-après, puis on plonge le ballon dans un bain d'huile à 125° ± 1°C. Dès le début de la précipitation du cotélomère (ce qui correspond à un très faible taux de conversion "α" en acrylamide), on arrête la réaction. On refroidit rapidement le mélange, puis le lave avec 100 ml d'acétone pour éliminer l'ester et l'acrylamide n'ayant pas réagi. On lave ensuite le précipité avec 100 ml d'éther anhydre afin de faciliter le séchage du produit. Après filtration et séchage à l'étuve à 80°C sous vide (20 torr), les cotélomères obtenus sont soumis à une analyse élémentaire du fluor, de l'azote, du carbone et de l'hydrogène.
Le tableau 2 suivant rassemble les résultats ainsi obtenus. Le degré de télomérisation moyen en nombre, $\bar{D}\bar{P}\bar{n}$, est calculé soit à partir de la teneur en fluor par la relation suivante :

$$\bar{D}\bar{P}\bar{n} = \frac{347,90}{\%\,F} - 7,17$$

soit à partir des teneurs en azote et en fluor par la relation suivante :

$$\bar{D}\bar{P}\bar{n} = \frac{247}{14} \times \frac{\%\,N}{\%\,F}$$

**EXEMPLE 13:**

On opère comme à l'exemple 12, sauf que :
a) on n'utilise que 0,7 g d'acrylamide et
b) on n'arrête pas la réaction, mais maintient à 125°C pendant 16 heures et demie.
Le tableau 3 suivant rassemble les résultats obtenus pour 5 opérations effectuées avec des quantités variables de catalyseur.

**TABLEAU 2**

| Co | % F | % N | % C | % H | $\overline{DPn}$ calculé par rapport à % F | % N/% F | α |
|---|---|---|---|---|---|---|---|
| $1.10^{-2}$ | 16,80 | 11,86 | 38,92 | 4,96 | 13,53 | 12,45 | 0,002 |
| $7{,}5.10^{-3}$ | 16,43 | 12,08 | 40,8 | 5,36 | 14,0 | 13,0 | 0,01 |
| $6{,}8.10^{-3}$ | 13,24 | 13,06 | 41,3 | 5,37 | 19,1 | 17,4 | 0,02 |
| $5.10^{-3}$ | 12,54 | 13,36 | 41,5 | 5,39 | 20,57 | 18,8 | 0,03 |
| $3{,}8.10^{-3}$ | 9,41 | 14,56 | 42,0 | 5,41 | 29,8 | 27,3 | 0,06 |
| $1.10^{-3}$ | 2,68 | 16,65 | 44,96 | 6,76 | 122,6 | 110 | 0,11 |
| $5.10^{-4}$ | 0,98 | 17,63 | 45,46 | 6,96 | 348 | 317 | 0,15 |
| $2{,}5.10^{-4}$ | 0,26 | 17,88 | 45,89 | 6,55 | 1331 | 1213 | 0,06 |

**TABLEAU 3**

| Co | % F | % N | $\overline{DPn}$ calculé par rapport à % F | % N/ % F | Rendement en % |
|---|---|---|---|---|---|
| $1.10^{-2}$ | 15,36 | 12,48 | 15,7 | 14,3 | 52,7 |
| $7{,}5.10^{-3}$ | 14,32 | 12,32 | 17,1 | 15,2 | 60,3 |
| $5.10^{-3}$ | 13,17 | 12,72 | 19,1 | 17 | 74,7 |
| $1.10^{-3}$ | 3,03 | 16,83 | 108 | 98 | 95,3 |
| $5.10^{-4}$ | 2,72 | 17,12 | 121 | 111 | 93,3 |

**Revendications** pour les etats contractants: BE, CH, DE, GB, IT, LI, LU, NL, SE

1. Télogènes fluorés à terminaison $CCl_3$, caractérisés en ce qu'ils répondent à la formule générale :

$$Rf - Q - CCl_3 \qquad (III)$$

dans laquelle Rf désigne un radical perfluoroalkyle, linéaire ou ramifié, contenant de 2 à 20 atomes de carbone et Q représente un groupe de liaison choisi parmi ceux de formules :

$$-(CH_2)_r-OCO-(CH_2)_s- \qquad (IV)$$

$$-(CH_2)_r-OCO-(CH_2)_s-\underset{\displaystyle X}{\underset{|}{C}}H-W- \qquad (V)$$

$$-(CH_2)_{r'}-SO2-\underset{\displaystyle R}{\underset{|}{N}}-CH_2CH_2-OCO-(CH_2)_s- \qquad (VI)$$

$$-(CH_2)_{r'}-SO_2-\underset{\displaystyle R}{\underset{|}{N}}-CH_2CH_2-OCO-(CH_2)_s-\underset{\displaystyle X}{\underset{|}{C}}H-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{\underset{|}{CH}}-W- \qquad (IX)$$

$$-(CH_2)_{r'}-COO-CH_2-\underset{OH}{\underset{|}{CH}}-CH_2- \qquad (X)$$

$$-(CH_2)_{r'}-\underset{X}{\underset{|}{CH}}-W- \qquad (XI)$$

$$-(CH_2)_{r'}-CONH-(CH_2)_{s'}-\underset{X}{\underset{|}{CH}}-W- \qquad (XII)$$

$$-(CH_2)_{r'}-SO_2NH-(CH_2)_{s'}-\underset{X}{\underset{|}{CH}}-W- \qquad (XIII)$$

$$-(CH_2)_{r}-Y-Z-\underset{X}{\underset{|}{CH}}-W- \qquad (XIV)$$

$$-O-\underset{X}{\underset{|}{CF}}-CF_2- \qquad (XV)$$

ou Rf et Q représentent ensemble un groupement de formule :

$$X-\left[\underset{ORf}{\underset{|}{CF}}-CF_2\right]_u-W'- \qquad (XVI)$$

ou

$$X-\left[\underset{CO-O(CH_2)_r-Rf}{\underset{|}{CR'}}-CH_2\right]_u-W'- \qquad (XVII)$$

dans lesquelles :

r et s' sont des nombres entiers allant de 1 à 12,

r' et s sont des nombres entiers allant de 0 à 12,

X représente un atome d'hydrogène, de chlore ou de brome,
W représente un pont $-CH_2-$, $-CH_2-CCl_2-$, $-CH_2-CCl_2-CH_2-$ ou $-CH_2CCl_2-CF_2-$,
R représente un atome d'hydrogène ou un groupe alkyle contenant de 1 à 4 atomes de carbone,
Y représente un atome d'oxygène ou de soufre,
Z représente un pont $-CH_2-$, $-CH_2-C_6H_4-$ ou $-COO-(CH_2)_t-$, t étant un nombre entier allant de 0 à 9,
R' représente un atome d'hydrogène ou un groupe méthyle,
W' représente une liaison directe ou un pont $-CCl_2-$, $-CCl_2-CH_2-$ ou $-CCl_2-CF_2-$ et,
u est un nombre allant de 2 à 100.

2. Télogènes selon la revendication 1, dans lesquels r est égal à 1 ou 2, r' est égal à 0 ou 2, s est égal à 0 ou 1, s' est égal à 1, X est un atome de chlore, W est un pont $-CH_2-$, R représente un atome d'hydrogène ou un groupe méthyle, Y est un atome d'oxygène et Z est un pont $-CH_2-$.

3. Télogènes selon la revendication 2 , dans lesquels Rf est un radical perfluoroalkyle linéaire contenant de 2 à 14 atomes de carbone et r est égal à 2.

4. Procédé de préparation des télogènes selon la revendication 1, caractérisé en ce que l'on introduit sur un composé fluoré une terminaison $CCl_3$ par :

a) estérification d'un alcool ou d'un acide fluoré au moyen d'un acide ou respectivement d'un alcool ou d'un époxyde contenant ladite terminaison $CCl_3$, ou

b) condensation d'un halogénure d'acide carboxylique ou sulfonique fluoré avec une amine contenant ladite terminaison $CCl_3$, ou

c) addition sur une oléfine fluorée d'un halogéno-alcane choisi parmi le tétrachlorure de carbone, le bromotrichlorométhane, le chloroforme, l'hexachloroéthane, le pentachloroéthane, l'hexachloro-1,1,1,3,3,3 propane et le difluoro-2,2 hexachloro-1,1,1,3,3,3 propane,

ou

d) condensation d'un alcool ou thiol fluoré avec le produit d'addition sur le chlorométhylstyrène d'un des halogéno-alcanes précités.

5. Utilisation des télogènes selon l'une des revendications 1 à 3, pour la fabrication de cotélomères biséquencés comportant une séquence totalement ou partiellement fluorée et une séquence polymérique compatible avec le milieu d'application final.

6. Utilisation selon la revendication 5, caractérisée en ce que l'on télomérise par catalyse rédox sur un télogène selon l'une des revendications 1 à 3 un ou plusieurs monomères éthyléniques de formule :

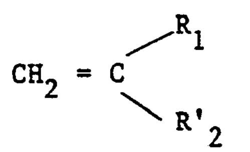

(XXXXI)

dans laquelle $R_1$ représente un atome d'hydrogène, de chlore ou de fluor ou un groupe cyano, méthyle ou carboxyméthyle, $R'_2$ représente un atome d'halogène, un groupe acyloxy, cyano, carboxy, ester carboxylique, alcoxyalkyle, sulfo ou carbamoyle, ou un radical pyrrolidone-2 yle-1, pyridyle-4 ou phényle éventuellement substitué par un groupe alkyle, chlorométhyle ou sulfo, $R_1$ ne peut être un groupe carboxyméthyle que si $R'_2$ est un groupe carboxy, et $R_1$ et $R'_2$ ne peuvent pas représenter simultanément un groupe cyano.

7. Utilisation selon la revendication 6, caractérisée en ce que le monomère est l'acrylamide.

8. Cotélomères biséquencés, caractérisés en ce qu'ils répondent à la formule générale :

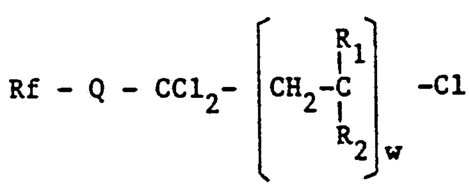

(XXXX)

dans laquelle Rf, Q et $R_1$ ont la même signification que dans les revendications 1 et 6, $R_2$ représente un atome d'halogène, un groupe hydroxy, cyano, carboxy, ester carboxylique, alcoxyalkyle, sulfo ou carbamoyle, ou un radical pyrolidone-2 yle-1, pyridyle-4 ou phényle éventuellement substitué par un groupe alkyle, chlorométhyle ou sulfo, $R_1$ ne peut représenter un groupe carboxyméthyle que si $R_2$ est un groupe carboxy, $R_1$ et $R_2$ ne peuvent pas représenter simultanément un groupe cyano, et w peut prendre toute valeur allant de 1 à 1500.

9. Cotélomères selon la revendication 8, dans lesquels Rf et Q correspondent aux définitions selon l'une des revendications 2 et 3, $R_1$ est un atome d'hydrogène et $R_2$ un groupe $CONH_2$.

**Revendications** pour l'etat contractnt: AT

1. Procédé de préparation de télogénes fluorés de formule générale :

$$Rf - Q - CCl_3 \qquad (III)$$

dans laquelle Rf désigne un radical perfluoroalkyle, linéaire ou ramifié, contenant de 2 à 20 atomes de carbone et Q représente un groupe de liaison choisi parmi ceux de formules :

$$-(CH_2)_r-OCO-(CH_2)_s- \qquad (IV)$$

$$-(CH_2)_r-OCO-(CH_2)_s-\underset{X}{\underset{|}{C}}H-W- \qquad (V)$$

$$-(CH_2)_{r'}-SO2-\underset{R}{\underset{|}{N}}-CH_2CH_2-OCO-(CH_2)_s- \qquad (VI)$$

$$-(CH_2)_{r'}-SO_2-\underset{R}{\underset{|}{N}}-CH_2CH_2-OCO-(CH_2)_s-\underset{X}{\underset{|}{C}}H-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{\underset{|}{C}}H-W- \qquad (IX)$$

$$-(CH_2)_{r'}-COO-CH_2-\underset{OH}{\underset{|}{C}}H-CH_2- \qquad (X)$$

$$-(CH_2)_{r'}-\underset{X}{\underset{|}{C}}H-W- \qquad (XI)$$

$$-(CH_2)_{r'}-CONH-(CH_2)_{s'}-\underset{X}{\underset{|}{C}}H-W- \qquad (XII)$$

$$-(CH_2)_{r'}-SO_2NH-(CH_2)_{s'}-\underset{X}{\underset{|}{C}}H-W- \qquad (XIII)$$

$$-(CH_2)_r-Y-Z-\underset{X}{\underset{|}{C}}H-W- \qquad (XIV)$$

$$-O-\underset{X}{\underset{|}{C}}F-CF_2- \qquad (XV)$$

ou Rf et Q représentent ensemble un groupement de formule :

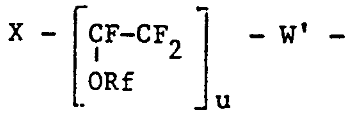

(XVI)

ou 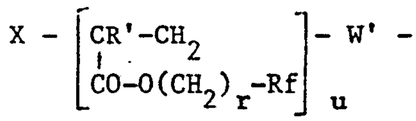

(XVII)

dans lesquelles :

r et s' sont des nombres entiers allant de 1 à 12,

r' et s sont des nombres entiers allant de 0 à 12,

X représente un atome d'hydrogène, de chlore ou de brome,

W représente un pont $-CH_2-$, $-CH_2-CCl_2-$, $-CH_2-CCl_2-CH_2-$ ou $-CH_2CCl_2-CF_2-$,

R représente un atome d'hydrogène ou un groupe alkyle contenant de 1 à 4 atomes de carbone,

Y représente un atome d'oxygène ou de soufre,

Z représente un pont $-CH_2-$, $-CH_2-C_6H_4-$ ou $-COO-(CH_2)_t-$, t étant un nombre entier allant de 0 à 9,

R' représente un atome d'hydrogène ou un groupe méthyle,

W' représente une liaison directe ou un pont $-CCl_2-$, $-CCl_2-CH_2-$ ou $-CCl_2-CF_2-$ et,

u est un nombre allant de 2 à 100, caractérisé en ce que l'on introduit sur un composé fluoré une terminaison $CCl_3$ par :

a) estérification d'un alcool ou d'un acide fluoré au moyen d'un acide ou respectivement d'un alcool ou d'un époxyde contenant ladite terminaison $CCl_3$, ou

b) condensation d'un halogénure d'acide carboxylique ou sulfonique fluoré avec une amine contenant ladite terminaison $CCl_3$, ou

c) addition sur une oléfine fluorée d'un halogéno-alcane choisi parmi le tétrachlorure de carbone, le bromotrichlorométhane, le chloroforme, l'hexachloroéthane, le pentachloroéthane, l'hexachloro-1,1,1,3,3,3 propane et le difluoro-2,2 hexachloro-1,1,1,3,3,3 propane, ou

d) condensation d'un alcool ou thiol fluoré avec le produit d'addition sur le chlorométhylstyrène d'un des halogéno-alcanes précités.

2 . Procédé selon la revendication 1 dans lequel r est égal à 1 ou 2, r' est égal à 0 ou 2, s est égal à 0 ou 1, s' est égal à 1, X est un atome de chlore, W est un pont $-CH_2-$, R représente un atome d'hydrogène ou un groupe méthyle, Y est un atome d'oxygène et Z est un pont $-CH_2-$.

3. Procédé selon la revendication 2, dans lequel Rf est un radical perfluoroalkyle linéaire contenant de 2 à 14 atomes de carbone et r est égal à 2.

4. Utilisation des télogènes fluorés tels que définis dans l'une des revendications 1 à 3 , pour la fabrication de cotélomères biséquencés comportant une séquence totalement ou partiellement fluorée et une séquence polymérique compatible avec le milieu d'application final.

5. Utilisation selon la revendication 4, caractérisée en ce que l'on télomérise par catalyse rédox sur un télogène fluoré tel que défini dans l'une des revendications 1 à 3 un ou plusieurs monomères éthyléniques de formule :

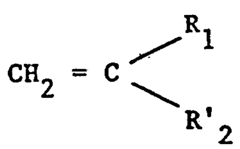

(XXXXI)

dans laquelle $R_1$ représente un atome d'hydrogène, de chlore ou de fluor ou un groupe cyano, méthyle ou carboxyméthyle, $R'_2$ représente un atome d'halogène, un groupe acyloxy, cyano, carboxy, ester carboxylique, alcoxyalkyle, sulfo ou carbamolyle, ou un radical pyrrolidone-2 yle-1, pyridyle-4 ou phényle éventuellement substitué par un groupe alkyle, chlorométhyle ou sulfo, $R_1$ ne peut être un groupe carboxyméthyle que si $R'_2$ est un groupe carboxy, et $R_1$ et $R'_2$ ne peuvent pas représenter simultanément un groupe cyano.

6. Utilisation selon la revendication 5, caractérisée en ce que le monomère est l'acrylamide.

**Patentansprüche** für die Vertragsstaaten: BE, CH, DE, GB, IT, LI, LU, NL, SE

1. Fluorierte Telogene mit der Endgruppe $CCl_3$, dadurch gekennzeichnet, daß sie der allgemeinen Formel

$$Rf - Q - CCl_3 \qquad (III)$$

entsprechen, in der Rf ein verzweigtes oder unverzweigtes Perfluoralkylradikal mit 2 bis 20 Kohlenstoffatomen bezeichnet und Q eine Brückengruppe entsprechend einer der folgenden Formeln bedeutet:

$$-(CH_2)_r-OCO-(CH_2)_s- \qquad (IV)$$

$$-(CH_2)_r-OCO-(CH_2)_s-\underset{X}{\underset{|}{CH}}-W- \qquad (V)$$

$$-(CH_2)_{r'}-SO_2-\underset{R}{\underset{|}{N}}-CH_2CH_2-OCO-(CH_2)_s- \qquad (VI)$$

$$-(CH_2)_{r'}-SO_2-\underset{R}{\underset{|}{N}}-CH_2CH_2-OCO-(CH_2)_s-\underset{X}{\underset{|}{CH}}-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{\underset{|}{CH}}-W- \qquad (IX)$$

$$-(CH_2)_{r'}-COO-CH_2-\underset{OH}{\underset{|}{CH}}-CH_2- \qquad (X)$$

$$-(CH_2)_{r'}-\underset{X}{\underset{|}{CH}}-W- \qquad (XI)$$

$$-(CH_2)_{r'}-CONH-(CH_2)_{s'}-\underset{X}{\underset{|}{CH}}-W- \qquad (XII)$$

$$-(CH_2)_{r'}-SO_2NH-(CH_2)_{s'}-\underset{X}{\underset{|}{C}}H-W- \qquad (XIII)$$

$$-(CH_2)_r-Y-Z-\underset{X}{\underset{|}{C}}H-W- \qquad (XIV)$$

$$-O-\underset{X}{\underset{|}{C}}F-CF_2- \qquad (XV)$$

oder Rf und Q zusammen eine Gruppe der Formel

$$X-\left[\begin{array}{l}CF-CF_2\\ |\\ ORf\end{array}\right]_u-W'- \qquad (XVI)$$

oder

$$X-\left[\begin{array}{l}CR'-CH_2\\ |\\ CO-O(CH_2)_r-Rf\end{array}\right]_u-W'- \qquad (XVII)$$

in denen
r und s' ganze Zahlen zwischen 1 und 12 sind,
r' und s ganze Zahlen zwischen 0 und 12,
X ein Wasserstoff-, Chlor- oder Bromatom bedeutet,
W eine der Brücken $-CH_2-$, $-CH_2-CCl_2$, $-CH_2-CCl_2-CH_2$ oder $-CH_2CCl_2-CF_2$
R ein Wasserstoffatom oder eine Alkylgruppe mit 1 bis 4 Kohlenstoffatomen,
Y ein Sauerstoff- oder Schwefelatom, Z für eine der Brücken $-CH_2-$, $-CH_2-C_6H_4-$ oder $-COO-(CH_2)_t-$ steht, wobei t eine ganze Zahl zwischen 0 und 9 ist,
R' ein Wasserstoffatom oder eine Methylgruppe darstellt,
W' eine Bindung oder eine der Brücken $-CCl_2-$, $-CCl_2-CH_2-$ oder $-CCl_2-CF_2$ und
u eine ganze Zahl zwischen 2 und 100 ist.

2. Telogene nach Patentanspruch 1, in denen r gleich 1 oder 2, r' gleich 0 oder 2 ist, s gleich 0 oder 1 und s' gleich 1, X ein Chloratom darstellt, W eine $-CH_2$-Brücke, R ein Wasserstoffatom oder eine Methylgruppe darstellt, Y ein Sauerstoffatom und Z eine $-CH_2$-Brücke.

3. Telogene nach Patentanspruch 2, in denen Rf ein unverzweigtes Perfluoralkylradikal mit 2 bis 14 Kohlenstoffatomen und r gleich 2 ist.

4. Verfahren zur Herstellung der Telogene nach Patentanspruch 1, dadurch gekennzeichnet, daß eine Endgruppe $CCl_3$ in eine fluorierte Verbindung eingeführt wird, und zwar durch

a) Veresterung eines fluorierten Alkohols oder einer fluorierten Säure mit einer die genannte Endgruppe $CCl_3$ enthaltenden Säure bzw. einem derartigen Alkohol oder Epoxid, durch

b) Kondensation eines fluorierten Carbon- oder Sulfonsäurehalogenids mit einem die genannte Endgruppe $CCl_3$ enthaltenden Amin, durch

c) Addition eines unter Tetrachlorkohlenstoff, Bromtrichlormethan, Chloroform, Hexachlorethan, Pentachlorethan, 1,1,1,3,3,3-Hexachlorpropan und 2,2-Difluor-1,1,1,3,3,3-hexachlorpropan gewählten Alkylhalogenids an ein fluoriertes Alken oder durch

d) Kondensation eines fluorierten Alkohols oder Thiols mit dem Additionsprodukt eines der oben genannten Alkylhalogenide an Chlormethylstyrol.

5. Verwendung der Telogene nach einem der Patentansprüche 1 bis 3 zur Herstellung von doppelkettigen Cotelomeren mit einer vollständig oder teilweise fluorierten Kette und einer dem Medium der Endanwendung entsprechenden Polymerkette.

6. Verwendung nach Patentanspruch 5, dadurch gekennzeichnet, daß durch Redoxkatalyse ein Telogen nach einem der Patentansprüche 1 bis 3 durch ein oder mehrere Ethylenmonomere der Formel

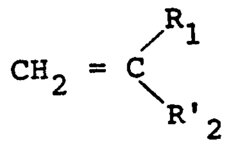 (XXXXI)

telomerisiert wird, in der $R_1$ ein Wasserstoff-, Chlor- oder Fluoratom oder eine Cyano-, Methyl-oder Carboxymethylgruppe darstellt und $R'_2$ ein Halogenatom, eine Acyloxy-, Cyan-, Carboxy-, Carbonsäureester-, Alkoxyalkyl-, Sulfo- oder Carbamoylgruppe, oder ein 2-Pyrrolidon-1-yl-, 4-Pyridyl- oder Phenylradikal, das eventuell durch eine Alkyl-, Chlormethyl- oder Sulfogruppe substituiert ist, wobei $R_1$ nur dann eine Carboxymethylgruppe sein kann, wenn $R'_2$ eine Carboxygruppe ist, und $R_1$ und $R'_2$ nicht gleichzeitig eine Cyangruppe darstellen können.

7. Verwendung der Telogene nach Patentanspruch 6, dadurch gekennzeichnet, daß das Monomer Acrylamid ist.

8. Doppelkettige Cotelomere, dadurch gekennzeichnet, daß sie der allgemeinen Formel

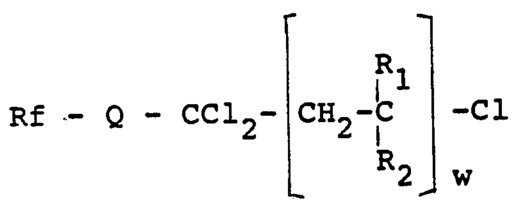 (XXXX)

entsprechen, in der Rf, Q und $R_1$ dieselbe Bedeutung haben, wie in den Patentansprüchen 1 und 6, $R_2$ ein Wasserstoffatom, eine Hydroxy-, Cyan-, Carboxy-, Carbonsäureester-, Alkoxyalkyl-, Sulfo-oder Carbamoylgruppe, oder ein 2-Pyrrolidon-1-yl-, 4-Pyridyl- oder Phenylradikal, das eventuell durch eine Alkyl-, Chlormethyl- oder Sulfogruppe substituiert ist, bedeutet, wobei $R_1$ nur dann eine Carboxymethylgruppe sein kann, wenn $R_2$ eine Carboxygruppe ist, und $R_1$ und $R_2$ nicht gleichzeitig eine Cyangruppe darstellen können, und w einen beliebigen Wert zwischen 1 und 1500 annehmen kann.

9. Cotelomere nach Patentanspruch 8, in denen Rf und Q den Definitionen eines der Patentansprüche 2 und 3 entsprechend, $R_1$ ein Wasserstoffatom und $R_2$ eine $CONH_2$-Gruppe bedeuten.

**Patentansprüche** für den Vertragsstaat: AT

1. Verfahren zur Herstellung fluorierter Telogene der allgemeinen Formel

$$Rf - Q - CCl_3 \qquad (III)$$

in der Rf ein verzweigtes oder unverzweigtes Perfluoralkylradikal mit 2 bis 20 Kohlenstoffatomen bezeichnet und Q eine Brückengruppe entsprechend einer der folgenden Formeln bedeutet:

$$-(CH_2)_r-OCO-(CH_2)_s- \qquad (IV)$$

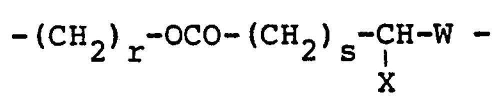 (V)

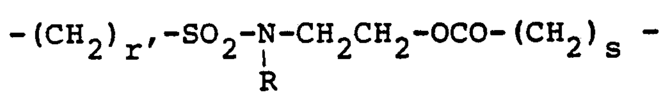 (VI)

$$-(CH_2)_{r'}-SO_2-\underset{R}{N}-CH_2CH_2-OCO-(CH_2)_s-\underset{X}{CH}-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{CH}-W- \qquad (IX)$$

$$-(CH_2)_{r'}-COO-CH_2-\underset{OH}{CH}-CH_2- \qquad (X)$$

$$-(CH_2)_{r'}-\underset{X}{CH}-W- \qquad (XI)$$

$$-(CH_2)_{r'}-CONH-(CH_2)_{s'}-\underset{X}{CH}-W- \qquad (XII)$$

$$-(CH_2)_{r'}-SO_2NH-(CH_2)_{s'}-\underset{X}{CH}-W- \qquad (XIII)$$

$$-(CH_2)_{r}-Y-Z-\underset{X}{CH}-W- \qquad (XIV)$$

$$-O-\underset{X}{CF}-CF_2- \qquad (XV)$$

oder Rf und Q zusammen eine Gruppe der Formel

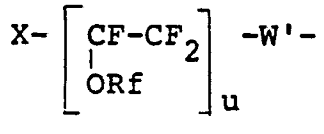 (XVI)

oder

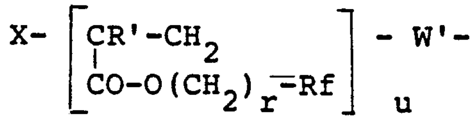 (XVII)

in denen
r und s' ganze Zahlen zwischen 1 und 12 sind,
r' und s ganze Zahlen zwischen 0 und 12,
X ein Wasserstoff-, Chlor- oder Bromatom bedeutet,
W eine der Brücken $-CH_2-$, $-CH_2-CCl_2$, $-CH_2-CCl_2-CH_2$ oder $-CH_2CCl_2-CF_2$
R ein Wasserstoffatom oder eine Alkylgruppe mit 1 bis 4 Kohlenstoffatomen,
Y ein Sauerstoff- oder Schwefelatom,
Z für eine der Brücken $-CH_2-$, $-CH_2-C_6H_4-$ oder $-COO-(CH_2)_t-$ steht, wobei t eine ganze Zahl zwischen 0 und 9 ist,
R' ein Wasserstoffatom oder eine Methylgruppe darstellt,
W' eine Bindung oder eine der Brücken $-CCl_2-$, $-CCl_2-CH_2-$ oder $-CCl_2-CF_2$ und
u eine ganze Zahl zwischen 2 und 100 ist, dadurch gekennzeichnet, daß eine Endgruppe $CCl_3$ in eine fluorierte Verbindung eingeführt wird, und zwar durch

a) Veresterung eines fluorierten Alkohols oder einer fluorierten Säure mit einer die genannte Endgruppe $CCl_3$ enthaltenden Säure bzw. einem derartigen Alkohol oder Epoxid, durch

b) Kondensation eines fluorierten Carbon- oder Sulfonsäurehalogenids mit einem die genannte Endgruppe $CCl_3$ enthaltenden Amin, durch

c) Addition eines unter Tetrachlorkohlenstoff, Bromtrichlormethan, Chloroform, Hexachlorethan, Pentachlorethan, 1,1,1,3,3,3-Hexachlorpropan und 2,2-Difluor-1,1,1,3,3,3-hexachlorpropan gewählten Alkylhalogenids an ein fluoriertes Alken oder durch

d) Kondensation eines fluorierten Alkohols oder Thiols mit dem Additionsprodukt eines der oben genannten Alkylhalogenide an Chlormethylstyrol.

2. Verfahren nach Patentanspruch 1, in dem r gleich 1 oder 2, r' gleich 0 oder 2 ist, s gleich 0 oder 1 und s' gleich 1, X ein Chloratom darstellt, W eine $-CH_2$-Brücke, R ein Wasserstoffatom oder eine Methylgruppe darstellt, Y ein Sauerstoffatom und Z eine $-CH_2$-Brücke.

3. Verfahren nach Patentanspruch 2, in dem Rf ein unverzweigtes Perfluoralkylradikal mit 2 bis 14 Kohlenstoffatomen und r gleich 2 ist.

4. Verwendung der in einem der Patentansprüche 1 bis 3 angegebenen Telogene zur Herstellung von doppelkettigen Cotelomeren mit einer vollständig oder teilweise fluorierten Kette und einer dem Medium der Endanwendung entsprechenden Polymerkette.

5. Verwendung nach Patentanspruch 4, dadurch gekennzeichnet, daß durch Redoxkatalyse eines der in einem der Patentansprüche 1 bis 3 angegebenen Telogene durch ein oder mehrere Ethylenmonomere der Formel

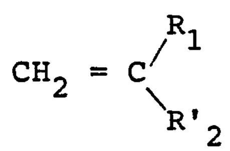 (XXXXI)

telomerisiert wird, in der $R_1$ ein Wasserstoff-, Chlor- oder Fluoratom oder eine Cyano-, Methyloder Carboxymethylgruppe darstellt und $R'_2$ ein Halogenatom, eine Acyloxy-, Cyan-, Carboxy-, Carbonsäureester-, Alkoxyalkyl-, Sulfo- oder Carbamoylgruppe, oder ein 2-Pyrrolidon-1-yl-, 4-Pyridyl- oder Phenylradikal, das eventuell durch eine Alkyl-, Chlormethyl- oder Sulfogruppe substituiert ist, wobei $R_1$ nur dann eine Carboxymethylgruppe sein kann, wenn $R'_2$ eine Carboxygruppe ist, und $R_1$ und $R'_2$ nicht gleichzeitig eine Cyangruppe darstellen können.

6. Verwendung der Telogene nach Patentanspruch 5, dadurch gekennzeichnet, daß das Monomer Acrylamid ist.

**Claims** for the contracting states: BE, CH, DE, GB, LT, LI, LU, NL, SE

1. Fluorinated telogens possessing a $CCl_3$ end group, characterised in that they correspond to the general formula:

$$Rf - Q - CCl_3 \qquad (III)$$

in which Rf denotes a linear or branched perfluoroalkyl radical containing from 2 to 20 carbon atoms and Q denotes a linkage group chosen from those of formulae:

$$-(CH_2)_r-OCO-(CH_2)_s- \qquad (IV)$$

$$-(CH_2)_r-OCO-(CH_2)_s-\underset{X}{\underset{|}{CH}}-W- \qquad (V)$$

$$-(CH_2)_{r'}-SO2-\underset{R}{\underset{|}{N}}-CH_2CH_2-OCO-(CH_2)_s- \qquad (VI)$$

$$-(CH_2)_{r'}-SO_2-\underset{R}{\underset{|}{N}}-CH_2CH_2-OCO-(CH_2)_s-\underset{X}{\underset{|}{CH}}-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{\underset{|}{CH}}-W- \qquad (IX)$$

$$-(CH_2)_{r'}-COO-CH_2-\underset{OH}{\underset{|}{CH}}-CH_2- \qquad (X)$$

$$-(CH_2)_{r'}-\underset{X}{\underset{|}{CH}}-W- \qquad (XI)$$

$$-(CH_2)_{r'}-CONH-(CH_2)_{s'}-\underset{X}{\underset{|}{CH}}-W- \qquad (XII)$$

$$-(CH_2)_{r'}-SO_2NH-(CH_2)_{s'}-\underset{X}{\underset{|}{CH}}-W- \qquad (XIII)$$

$$-(CH_2)_r-Y-Z-\underset{X}{\underset{|}{CH}}-W- \qquad (XIV)$$

$$-O-\underset{X}{\underset{|}{CF}}-CF_2- \qquad (XV)$$

or Rf and Q denote together a group of formula:

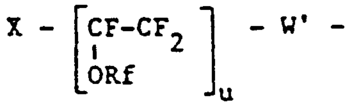

(XVI)

or

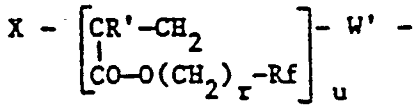

(XVII)

in which:

r and s' are integers ranging from 1 to 12,

r' and s are integers ranging from 0 to 12,

X denotes a hydrogen, chlorine or bromine atom,

X denotes a $-CH_2-$, $-CH_2-CCl_2-$, $-CH_2-CCl_2-CH_2-$ or $-CH_2CCl_2-CF_2-$ bridge,

R denotes a hydrogen atom or an alkyl group containing from 1 to 4 carbon atoms,

Y denotes an oxygen or sulphur atom,

Z denotes a $-CH_2-$, $-CH_2-C_6H_4-$ or $-COO-(CH_2)_t-$ bridge, t being an integer ranging from 0 to 9,

R' denotes a hydrogen atom or a methyl group,

W' denotes a direct bond or a $-CCl_2-$, $-CCl_2-CH_2-$ or $-CCl_2-CF_2-$ bridge, and

u is a number ranging from 2 to 100.

2. Telogens according to Claim 1, in which r equals 1 or 2, r' equals 0 or 2, s equals 0 or 1, s' equals 1, X is a chlorine atom, W is a $-CH_2-$ bridge, R denotes a hydrogen atom or a methyl group, Y is an oxygen atom and Z is a $-CH_2-$ bridge.

3. Telogens according to Claim 2, in which Rf is a linear perfluoroalkyl radical containing from 2 to 14 carbon atoms and r equals 2.

4. Process for preparing the telogens according to Claim 1, characterised in that a $CCl_3$ end group is introduced onto a fluorinated compound by:

a) esterification of a fluorinated alcohol or acid with, respectively an acid or an alcohol or epoxide containing the said $CCl_3$ end group, or

b) condensation of a fluorinated carboxylic or sulphonic acid halide with an amine containing the said $CCl_3$ end group, or

c) addition to a fluorinated olefin of a haloalkane chosen from carbon tetrachloride, bromotrichloromethane, chloroform, hexachloroethane, pentachloroethane, 1,1,1,3,3,3-hexachloropropane and 2,2-difluoro-1,1,1,3,3,3-hexachloropropane, or

d) condensation of a fluorinated alcohol or thiol with the product of addition to chloromethylstyrene of one of the abovementioned haloalkanes.

5. Use of the telogens according to one of Claims 1 to 3 for manufacturing double-sequence cotelomers containing a completely or partially fluorinated sequence and a polymeric sequence which is compatible with the final application medium.

6. Use according to Claim 5, characterised in that there is/are telomerised, by redox catalysis with a telogen according to one of Claims 1 to 3, one or more ethylenic monomers of formula:

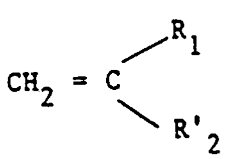

(XXXXI)

in which $R_1$ denotes a hydrogen, chlorine or fluorine atom or a cyano, methyl or carboxymethyl group, $R'_2$ denotes a halogen atom, an acyloxy, cyano, carboxy, carboxylic ester, alkoxyalkyl, sulpho or carbamoyl group, or a 2-pyrrolidon-1-yl, 4-pyridyl or phenyl radical optionally substituted with an alkyl, chloromethyl or sulpho group, $R_1$ can only be a carboxymethyl group if $R'_2$ is a carboxy group, and $R_1$ and $R'_2$ cannot simultaneously denote a cyano group.

7. Use according to Claim 6, characterised in that the monomer is acrylamide.

8. Double-sequence cotelomers, characterised in that they correspond to the general formula:

$$Rf - Q - CCl_2 - \left[ CH_2 - \overset{R_1}{\underset{R_2}{C}} \right]_w - Cl \qquad (XXXX)$$

in which Rf, Q and $R_1$ have the same meaning as in Claims 1 and 6, $R_2$ denotes a halogen atom, a hydroxy, cyano, carboxy, carboxylic ester, alkoxyalkyl, sulpho or carbamoyl group, or a 2-pyrrolidon-1-yl, 4-pyridyl or phenyl radical optionally substituted with an alkyl, chloromethyl or sulpho group, $R_1$ can only denote a carboxymethyl group if $R_2$ is a carboxy group, $R_1$ and $R_2$ cannot simultaneously denote a cyano group and w can assume any value ranging from 1 to 1500.

9. Cotelomers according to Claim 8, in which Rf and Q correspond to the definitions according to one of Claims 2 and 3, $R_1$ is a hydrogen atom and $R_2$ a $CONH_2$ group.

**Claims** for the contracting state: AT

1. Process for the preparation of fluorinated telogens of general formula:

$$Rf - Q - CCl_3 \qquad (III)$$

in which Rf denotes a linear or branched perfluoroalkyl radical containing from 2 to 20 carbon atoms and Q denotes a linkage group chosen from those of formulae:

$$-(CH_2)_r-OCO-(CH_2)_s- \qquad (IV)$$

$$-(CH_2)_r-OCO-(CH_2)_s-\underset{X}{CH}-W- \qquad (V)$$

$$-(CH_2)_{r'}-SO2-\underset{R}{N}-CH_2CH_2-OCO-(CH_2)_s- \qquad (VI)$$

$$-(CH_2)_{r'}-SO_2-\underset{R}{N}-CH_2CH_2-OCO-(CH_2)_s-\underset{X}{CH}-W- \qquad (VII)$$

$$-(CH_2)_{r'}-COO-(CH_2)_{s'}- \qquad (VIII)$$

$$-(CH_2)_{r'}-COO-(CH_2)_{s'}-\underset{X}{CH}-W- \qquad (IX)$$

$$-(CH_2)_{r'}-COO-CH_2-\underset{OH}{CH}-CH_2- \qquad (X)$$

$$-(CH_2)_{r'}-\underset{X}{CH}-W- \qquad (XI)$$

$$-(CH_2)_{r'}-CONH-(CH_2)_{s'}-\underset{X}{\underset{|}{CH}}-W- \qquad (XII)$$

$$-(CH_2)_{r'}-SO_2NH-(CH_2)_{s'}-\underset{X}{\underset{|}{CH}}-W- \qquad (XIII)$$

$$-(CH_2)_{r}-Y-Z-\underset{X}{\underset{|}{CH}}-W- \qquad (XIV)$$

$$-O-\underset{X}{\underset{|}{CF}}-CF_2- \qquad (XV)$$

or Rf and Q denote together a group of formula:

$$X-\left[\underset{ORf}{\underset{|}{CF}}-CF_2\right]_u-W'- \qquad (XVI)$$

or

$$X-\left[\underset{CO-O(CH_2)_r-Rf}{\underset{|}{CR'}}-CH_2\right]_u-W'- \qquad (XVII)$$

in which:

r and s' are integers ranging from 1 to 12,
r' and s are integers ranging from 0 to 12,
X denotes a hydrogen, chlorine or bromine atom,
W denotes a $-CH_2-$, $-CH_2-CCl_2-$, $-CH_2-CCl_2-CH_2-$ or $-CH_2CCl_2-CF_2-$ bridge,
R denotes a hydrogen atom or an alkyl group containing from 1 to 4 carbon atoms,
Y denotes an oxygen or sulphur atom,
Z denotes a $-CH_2-$, $-CH_2-C_6H_4-$ or $-COO-(CH_2)_t-$ bridge, t being an integer ranging from 0 to 9,
R' denotes a hydrogen atom or a methyl group,
W' denotes a direct bond or a $-CCl_2-$, $-CCl_2-CH_2-$ or $-CCl_2-CF_2-$ bridge, and
u is a number ranging from 2 to 100, characterised in that a $CCl_3$ end group is introduced onto a fluorinated compound by:

a) esterification of a fluorinated alcohol or acid with, respectively an acid or an alcohol or epoxide containing the said $CCl_3$ end group, or

b) condensation of a fluorinated carboxylic or sulphonic acid halide with an amine containing the said $CCl_3$ end group, or c) addition to a fluorinated olefin of a haloalkane chosen from carbon tetrachloride, bromotrichloromethane, chloroform, hexachloroethane, pentachloroethane, 1,1,1,3,3,3-hexachloropropane and 2,2-difluoro-1,1,1,3,3,3-hexachloropropane, or

d) condensation of a fluorinated alcohol or thiol with the product of addition to chloromethylstyrene of one of the abovementioned haloalkanes.

2. Process according to Claim 1, in which r equals 1 or 2, r' equals 0 or 2, s equals 0 or 1, s' equals 1, X is a chlorine atom, W is a $-CH_2-$ bridge, R denotes a hydrogen atom or a methyl group, Y is an oxygen atom and Z is a $-CH_2-$ bridge.

3. Process according to Claim 2, in which Rf is a linear perfluoroalkyl radical containing from 2 to 14 carbon atoms and r equals 2.

4. Use of the fluorinated telogens as defined in one of Claims 1 to 3 for manufacturing double-sequence cotelomers containing a completely or partially fluorinated sequence and a polymeric sequence which is compatible with the final application medium.

5. Use according to Claim 4, characterised in that there is/are telomerised, by redox catalysis with a fluorinated telogen as defined in one of Claims 1 to 3, one or more ethylenic monomers of formula:

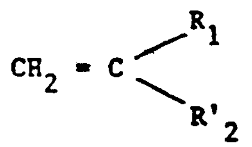

(XXXXI)

in which $R_1$ denotes a hydrogen, chlorine or fluorine atom or a cyano, methyl or carboxymethyl group, $R'_2$ denotes a halogen atom, an acyloxy, cyano, carboxy, carboxylic ester, alkoxyalkyl, sulpho or carbamoyl group, or a 2-pyrrolidon-1-yl, 4-pyridyl or phenyl radical optionally substituted with an alkyl, chloromethyl or sulpho group, $R_1$ can only be a carboxymethyl group if $R'_2$ is a carboxy group, and $R_1$ and $R'_2$ cannot simultaneously denote a cyano group.

6. Use according to Claim 5, characterised in that the monomer is acrylamide.